# FASCICULE DE BREVET EUROPEEN

(11) **EP 0 623 341 B1**
(45) Date de publication et mention de la délivrance du brevet: **16.10.1996**
(21) Numéro de dépôt: 94400984.4
(22) Date de dépôt: 05.05.1994
(51) Int. Cl.: A61K 7/48

(54) **Utilisation de dérivés de la 4-thiorésorcine dans des compositions cosmétiques ou dermopharmaceutiques à action dépigmentante**
Verwendung von 4-Thiuoresorcin-Derivaten in kosmetischen oder dermopharmazeutischen Zusammensetzungen zur Hautdepigmentierung
Use of 4-thioresorcin derivatives in skin whitening cosmetic or dermopharmaceutical compositions

(30) Priorité: 06.05.1993 FR 9305452
(43) Date de publication de la demande: 09.11.1994
(73) Titulaire: L'OREAL, F-75008 Paris (FR)
(72) Inventeur: Lagrange, Alain, F-77700 Coupvray (FR); Borowiak, Hervé, F-93270 Sevran (FR)
(74) Mandataire: Stalla-Bourdillon, Bernard

(56) Documents cités:
- EP-A- 0 341 664
- CHEMICAL ABSTRACTS, vol. 118, no. 22, 31 Mai 1993, Columbus, Ohio, US; abstract no. 219474w, T. HAMAZAKI 'cosmetic containing resorcinols and Ir ray-blocking agents'
- DATABASE WPI Week 9125, Derwent Publications Ltd., London, GB; AN 91-181411 & JP-A-3 109 319 (NARIS KESHOHIN KK) 9 Mai 1991

## Description

La présente invention a pour objet l'utilisation de dérivés de la 4-thiorésorcine ou 4-thio 1,3 dihydroxybenzène dans des compositions cosmétiques ou dermatologiques par application topique dans le but de blanchir la peau ou de traiter les taches pigmentaires.

On rappellera que le mécanisme de formation de la pigmentation de la peau, c'est-à-dire de la formation des mélanines est particulièrement complexe et fait intervenir schématiquement les principales étapes suivantes :
Tyrosine → Dopa → Dopaquinone → Dopachrome → Mélanines
l'enzyme intervenant dans cette suite de réactions étant essentiellement la tyrosinase.

Les substances les plus utilisées à l'heure actuelle en tant que dépigmentants sont plus particulièrement l'hydroquinone et ses dérivés en particulier ses éthers tels que le monométhyléther d'hydroquinone.

Ces composés, s'ils ont une efficacité certaine, ne sont malheureusement pas exempts d'effets secondaires, ce qui peut rendre leur emploi délicat voir dangereux.

Ainsi l'hydroquinone, dont l'emploi est d'ailleurs limité à une concentration de 2 %, est un composé particulièrement irritant et cytotoxique pour le mélanocyte, dont le remplacement, total ou partiel a été envisagé par de nombreux auteurs.

Il a ainsi été proposé dans le brevet US n° 4 526 179 certains esters gras d'hydroquinone ayant une bonne activité et étant moins irritants et plus stables que l'hydroquinone.

De même on a proposé d'autres dérivés d'hydroquinone dans la demande japonaise n° 27909/86 ne présentant pas les inconvénients de l'hydroquinone mais dont l'efficacité s'est révélée relativement médiocre.

Il est bien établi qu'une substance exerce une action dépigmentante si elle agit directement sur la vitalité des mélanocytes épidermiques où se déroule normalement la mélanogénèse, et/ou si elle interfère avec une des étapes de la biosynthèse des mélanines soit en inhibant un des enzymes impliqués soit en s'intercalant comme analogue structural dans la voie de synthèse qui peut ainsi être bloquée d'où l'effet dépigmentant.

En application de ce principe, il a ainsi été proposé l'utilisation de composés tels que des 4-alkyle résorcines dans la demande de brevet EP 0 341 664.

L'utilisation de substances dépigmentantes topiques présentant une bonne efficacité et étant inoffensives, est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou secondaire à la contraception oestro-progestative, les hyperpigmentations localisées par hyperactivité et prolifération mélanocytaire bénigne telles que les taches pigmentaires séniles dites lentigo actiniques, les hyperpigmentations accidentelles telles que la photosensibilisation et la cicatrisation post-lésionnelle, ainsi que certaines leucodermies telles que le vitiligo où, à défaut de pouvoir repigmenter la peau lésée, on achève de dépigmenter les zones de peau normale résiduelle pour donner à l'ensemble de la peau une teinte blanche homogène.

Après de nombreuses études sur différentes substances, on a maintenant constaté de façon très surprenante que d'autres types de dérivés de résorcines, en particulier les dérivés de la 4-thio résorcine, portant divers substituants sur l'atome de soufre, avaient également une excellente action dépigmentante qui, pour la plupart, s'est avérée être supérieure à celle de l'hydroquinone dans le test d'inhibition "in vitro" de l'activité tyrosinase comme ceci sera décrit ci-après.

La présente invention a donc pour objet l'utilisation, à titre de composés actifs, de dérivés de la 4-thio résorcine pour la préparation d'une composition cosmétique ou dermatologique ayant une action dépigmentante les dits dérivés répondant à la formule générale suivante : 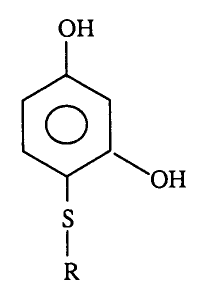
dans laquelle :
R représente un radical alkyle en C₁-C₆, un radical halogénoalkyle en C₁-C₆, un radical aryle, un radical aralkyle substitué ou non par un radical alkyle inférieur, un alcoxy inférieur ou un atome d'halogène, le radical -CₙH₂ₙ-COOR' où n est un nombre entier compris entre 1 et 5 et R' représente un radical alkyle en C₁-C₆, un radical morpholino-alkyle ou pipéridino-alkyle dont le radical alkyle est en C₁-C₆, ou le radical

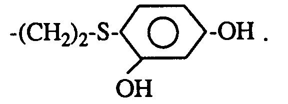

Selon l'invention, l'atome d'halogène est choisi de préférence parmi le chlore et le fluor.

Le radical aryle est de préférence le radical phényle et le radical aralkyle le radical benzyle.

Parmi les composés de formule (I), on peut citer notamment les suivants :
- la 4-méthyl thio résorcine,
- la 4-éthyl thio résorcine,
- La 4-n-butyl thio résorcine,
- la 4-n-propyl thio résorcine,
- la 4-isopropyl thio résorcine,
- la 4-éthoxycarbonyl méthyl thio résorcine,
- la 4-éthoxycarbonyl isopropyl thio résorcine,
- la 4-benzyl thio résorcine,
- la 4-phényl thio résorcine,
- la 4-(4'-méthoxy benzyl) thio résorcine,
- la 4-(4'-chloro benzyl) thio résorcine,
- la 4-(4'-méthyl benzyl) thio résorcine,
- la 4-(3'-morpholino propyl) thio résorcine,
- la 4-(4'-morpholino butyl) thio résorcine,
- la 4-(4'-pipéridino butyl) thio résorcine,
- la 4-(4'-chloro butyl) thio résorcine,
- la 4-(2',2'-dichloro 1',1',2'-trifluoroéthyl) thio résorcine, et
- le 1,2 bis-(2',4'-dihydroxyphényl) éthylène dithioéther.

Parmi les composés particulièrement préférés, on peut citer notamment :
- la 4-méthyl thio résorcine,
- la 4-éthyl thio résorcine,
- la 4-n-propyl thio résorcine,
- la 4-isopropyl thio résorcine,
- la 4-benzyl thio résorcine,
- le 1,2 bis-(2',4'-dihydroxyphényl) éthylène dithioéther,
- la 4-(3'-morpholino propyl) thio résorcine, et
- la 4-(éthoxy carbonyl méthyl) thio résorcine.

Dans les compositions dépigmentantes selon l'invention, la concentration en composés de formule (I) est généralement comprise entre 0,01 % et 10 % et de préférence entre 0,5 % et 5 % en poids par rapport au poids total de la composition.

Le véhicule des compositions peut être notamment une solution aqueuse ou hydroalcoolique, une émulsion du type huile-dans-l'eau ou eau-dans-l'huile, un gel émulsionné ou encore un système biphasé.

De préférence les compositions selon l'invention se présentent sous forme d'une lotion, d'une crème, d'un lait, d'un gel, d'un masque, de microsphères ou nanosphères ou de dispersions vésiculaires. Dans le cas des dispersions vésiculaires, les lipides constitutifs des vésicules peuvent être du type ionique ou non ionique ou bien un mélange de ceux-ci.

Ces compositions cosmétiques peuvent également contenir un humectant, un agent de surface, un kératolytique, un agent anti-inflammatoire, un agent complexant, un anti-oxydant, un conservateur, un parfum ou un filtre solaire.

Ces compositions sont appliquées par voie topique en quantité correspondant aux doses d'application usuelles pour le type de composition considéré (gel, crème, lotion, etc...). Par exemple dans le cas d'une crème on utilise de 0,5 à 3 mg et notamment de 1 à 2 mg de crème par cm2 de peau et par application, à raison d'une ou deux applications par jour.

Les composés de formule générale (I) sont connus, à l'exception de la 4-isopropyl thio résorcine, du 1,2-bis (2',4'-dihydroxyphényl) éthylène dithioéther et de la 4-(3-morpholino propyl) thio résorcine et sont obtenus selon les méthodes de synthèse conventionnelles. Les composés nouveaux mentionnés ci-dessus peuvent être préparés par ouverture de la tioxolone, en milieu basique, et alkylation par l'halogénure correspondant.

Leur procédé de préparation est décrit plus en détail, ci-après.

### Etudes "in vitro"

Certains des composés de formule générale (I) ont été étudiés comparativement à l'hydroquinone à quantité molaire équivalente dans le test d'inhibition in vitro de l'activité de la tyrosinase.

Selon ce test on suit par spectrométrie visible à 475 nm la quantité de dopachrome formée au cours de la chaîne de réactions de transformation de la tyrosine en mélanines. Ces réactions sont catalysées in vitro par la tyrosinase de champignons, en présence d'un co-substrat réducteur (par exemple la L-dopa en petite quantité) pour initier la réaction d'hydroxylation de la L-tyrosine en L-dopa, laquelle est ensuite oxydée catalytiquement en dopaquinone puis en dopachrome, produit intermédiaire avant les réactions d'oxydation non enzymatiques aboutissant à la formation des mélanines.

On mesure donc la concentration en dopachrome formé au cours du temps en présence et en absence de l'inhibiteur.

Les concentrations en inhibiteurs sont fixées à 50 % molaire par rapport à la concentration en tyrosine dans le milieu réactionnel.

On exprime l'effet d'inhibition par l'abaissement de la quantité maximale de dopachrome formée (valeur de densité optique à 475 nm lue au plateau de la courbe) par rapport à la quantité obtenue en l'absence d'inhibiteur.

### Protocole expérimental

### Réactifs :

A - Tampon phosphate 0,1 M pH = 6,5 (Tween 20 à 1 %)
B - Solution mère de L-tyrosine à 2.10⁻³ M dans A
C - Solution mère de L-dopa à 10⁻⁴ M dans A
D - Solution mère de tyrosinase de champignons à 2.400 unités/ml dans A
E - Solution mère de l'inhibiteur à 10⁻² M dans A (les solutions C et D sont à préparer le jour même)

### Résultats :

- cuve de référence : 3 ml de A
- cuve d'essai : 1 ml de B
   0,1 ml de C
   1,85ml de A + E
- homogénéiser et équilibrer à 25°C
- ajouter 0,05 ml de D
- mélanger rapidement et observer la cinétique par la mesure de l'absorbance à 475 nm en fonction du temps.

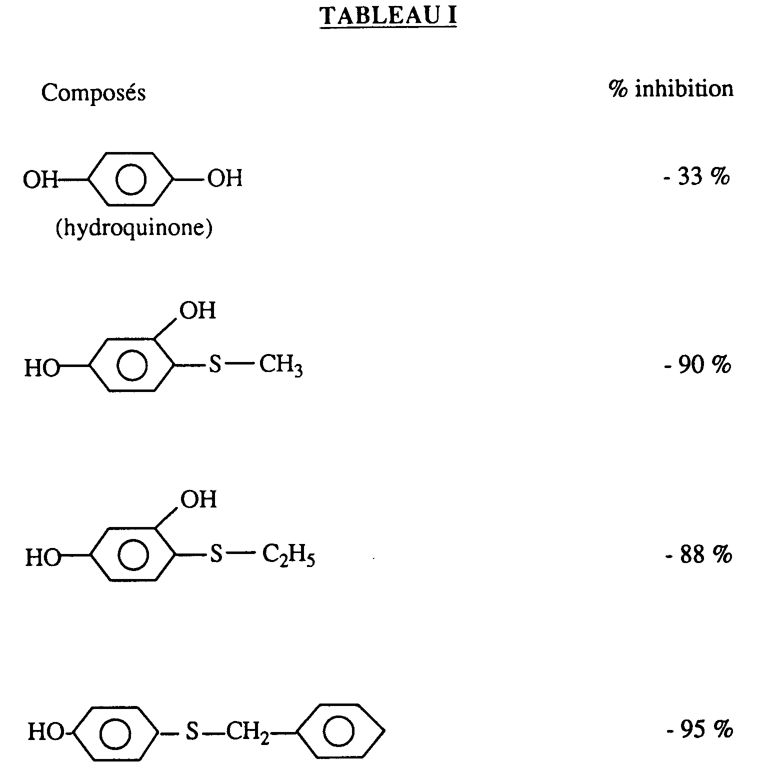

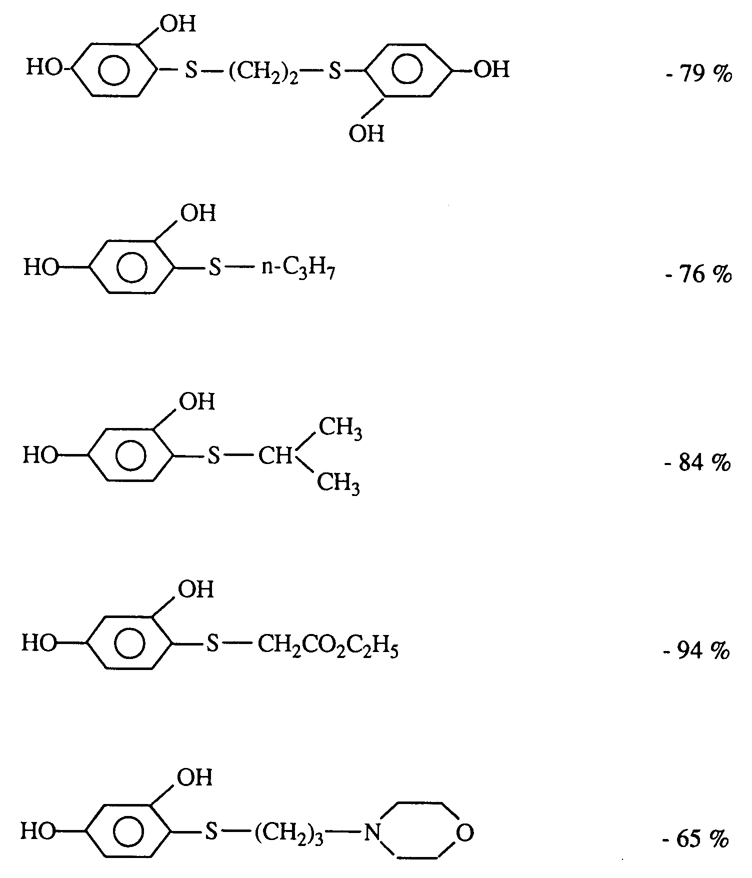

### EXEMPLES DE PREPARATION DES COMPOSES

### EXEMPLE 1 : Préparation de la 4-isopropyl thio résorcine

A une solution aqueuse de soude (3,5 eq) (6,7 g de soude dans 84 ml d'eau) à la température ambiante, on ajoute 8,4 g de tioxolone (1 eq). Après dissolution totale (30 minutes), on ajoute 5,5 ml d'iodure d'isopropyle. Après deux heures d'agitation à la température ambiante, on acidifie avec de l'acide chlorhydrique puis extrait avec de l'éther éthylique. Les phases organiques sont lavées, séchées et évaporées. Le résidu est recristallisé dans un mélange alcool/eau pour obtenir des cristaux jaune pale ayant un point de fusion de : 67°C.

| Analyse élémentaire : C₉ H₁₂ O₂ S | | | | |
|---|---|---|---|---|
| | C % | H % | O % | S % |
| Calculé | 58,67 | 6,56 | 17,37 | 17,4 |
| Trouvé | 59,58 | 6,62 | 17,43 | 16,52 |

### EXEMPLE 2 : Préparation du 1,2-bis(2',4'-dihydroxyphényl) éthylène dithioéther

On utilise le même mode opératoire qu'à l'Exemple 1 à partir de :
- 33,6 g: de tioxolone,
- 26,8 g: de soude en pastille,
- 335 ml: d'eau, et
- 18,8 g: de dibromo-1,2-éthane.

Après cristallisation dans le mélange alcool/eau, on obtient des cristaux beiges foncés de point de fusion : 164°C.

| Analyse élémentaire : C₁₄ H₁₄ O₄ S₂ | | | | |
|---|---|---|---|---|
| | C % | H % | O % | S % |
| Calculé | 54,17 | 4,54 | 20,62 | 20,66 |
| Trouvé | 53,71 | 5,11 | 21,03 | 20,11 |

### EXEMPLE 3 : Préparation de la 4-(3-morpholino propyl) thio résorcine

### (1) Préparation de la 4-(3-chloropropyl) thio résorcine

On utilise le même mode opératoire qu'à l'Exemple 1 à partir de :
- 16,8 g: de tioxolone,
- 33,5 ml: de soude 10N
- 135 ml: d'eau, et
- 17,3 g: de 1-bromo-3-chloro-propane.

On obtient une huile jaune pale que l'on utilise directement pour l'étape suivante.

| Analyse élémentaire : C₉ H₁₁ Cl O₂ S₂ | | | |
|---|---|---|---|
| | C % | H % | S % |
| Calculé | 49,13 | 5,07 | 14,66 |
| Trouvé | 48,96 | 4,92 | 14,28 |

### (2) Préparation de la 4-(3-morpholino propyl) thio résorcine

On chauffe 4 g de 4-(3-chloro-propyl) thio résorcine et 8 ml de morpholine pendant deux heures à 100°C. On verse dans de l'eau glacée et on extrait avec de l'acétate d'éthyle. Les phases organiques sont lavées, séchées et concentrées sous vide. L'huile jaune est cristallisée dans l'acétate d'éthyle pour conduire à des cristaux blancs ayant un point de fusion de : 99°C.

| Analyse élémentaire : C₁₃ H₁₉ N O₃ S | | | | | |
|---|---|---|---|---|---|
| | C % | H % | N % | O % | S % |
| Calculé | 57,97 | 7,11 | 5,2 | 17,82 | 11,9 |
| Trouvé | 57,59 | 6,75 | 5,08 | 18,53 | 11,76 |

### EXEMPLES DE COMPOSITION

### EXEMPLE 1 : Crème dépigmentante

| | |
|---|---|
| - 4-méthyl thio résorcine | 2,8 g |
| - Alcool cétylstéarylique oxyéthyléné à 20 moles d'oxyde d'éthylène | 1 g |
| - Monostéarate de glycol | 3 g |
| - Mélange de caprylate et caprate de coprah | 5 g |
| - Polymère d'acide acrylique réticulé vendu sous la dénomination de "Carbomer 934P" par la Société Goodrich | 0,3 g |
| - Triéthanolamine | 0,9 g |
| - Ethanol | 20 g |
| - Glycérine | 3 g |
| - Parfum, conservateurs q.s. | |
| - Eau q.s.p. | 100 g |

Dans cet exemple, on peut remplacer la 4-méthyl thio résorcine par 2,0 g de 1,2-bis(2',4'-dihydroxyphényl) éthylène dithioéther.

### EXEMPLE 2 : Lotion dépigmentante

| | |
|---|---|
| - 4-méthyl thio résorcine | 2,8 g |
| - Ethanol | 50 g |
| - Polyéthylèneglycol 400 | 30 g |
| - Ethoxydiglycol | 5 g |
| - Glycérine | 5 g |
| - Eau q.s.p. | 100 g |

Dans cet exemple, on peut remplacer la 4-méthyl thio résorcine par 2,5 g de 4-benzyl thio résorcine.

### EXEMPLE 3 : Crème dépigmentante

| | |
|---|---|
| - 4-méthyl thio résorcine | 2,5 g |
| - Glycérine | 5,0 g |
| - Diméthylpolysiloxane cyclique | 20 g |
| - Mélange de polycétyldiméthylsiloxane oxyéthyléné et oxypropyléné, d'isostéarate de polyglycéryle à 4 moles de glycérol et de laurate d'hexyle vendu sous la dénomination de "Abil W09" par la Société Goldschmidt | 3,0 g |
| - Parfum, conservateurs q.s. | |
| - Eau q.s.p. | 100 g |

Dans cet exemple, la 4-méthyl thio résorcine peut être remplacée par 1,8 g de 4-(3-morpholino propyl) thio résorcine.

## Revendications

1. Utilisation, à titre de composés actifs, de dérivés de résorcine pour la préparation d'une composition cosmétique ou dermatologique ayant une action dépigmentante, caractérisée par le fait que les dits dérivés répondent à la formule suivante : 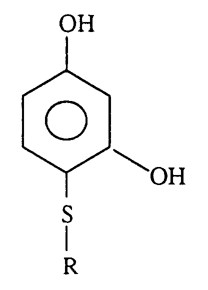 dans laquelle :
R représente un radical alkyle en C₁-C₆, un radical halogénoalkyle en C₁-C₆, un radical aryle, un radical aralkyle substitué ou non par un radical alkyle inférieur, un alcoxy inférieur ou un atome d'halogène, le radical -CₙH₂ₙ-COOR' où n est un nombre entier compris entre 1 et 5 et R' représente un radical alkyle en C₁-C₆, un radical morpholino-alkyle ou piperidino-alkyle dont le radical alkyle est en C₁-C₆, ou le radical

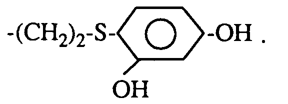

2. Utilisation selon la revendication 1, caractérisée par le fait que ledit radical aryle est le radical phényle.

3. Utilisation selon la revendication 1, caractérisée par le fait que ledit radical aralkyle est le radical benzyle.

4. Utilisation selon l'une quelconque des revendications précédentes, caractérisée par le fait que lesdits dérivés de formule (I) sont choisis dans le groupe constitué par :
- la 4-méthyl thio résorcine,
- la 4-éthyl thio résorcine,
- la 4-n-butyl thio résorcine,
- la 4-n-propyl thio résorcine,
- la 4-isopropyl thio résorcine,
- la 4-éthoxycarbonyl méthyl thio résorcine,
- la 4-éthoxycarbonyl isopropyl thio résorcine,
- la 4-benzyl thio résorcine,
- la 4-phényl thio résorcine,
- la 4-(4'-méthoxy benzyl) thio résorcine,
- la 4-(4'-chloro benzyl) thio résorcine,
- la 4-(4'-méthyl benzyl) thio résorcine,
- la 4-(3'-morpholino propyl) thio résorcine,
- la 4-(4'-morpholino butyl) thio résorcine,
- la 4-(4'-pipéridino butyl) thio résorcine,
- la 4-(4'-chloro butyl) thio résorcine,
- la 4-(2',2'-dichloro 1',1',2'-trifluoroéthyl) thio résorcine, et
- le 1,2 bis-(2',4'-dihydroxyphényl) éthylène dithioéther.

5. Utilisation selon l'une quelconque des revendications précédentes caractérisée par le fait que lesdits dérivés de Formule (I) sont choisis dans le groupe constitué par :
- la 4-méthyl thio résorcine,
- la 4-éthyl thio résorcine,
- la 4-n-propyl thio résorcine,
- la 4-isopropyl thio résorcine,
- la 4-benzyl thio résorcine,
- le 1,2 bis-(2',4'-dihydroxyphényl) éthylène dithioéther,
- la 4-(3'-morpholino propyl) thio résorcine, et
- la 4-(éthoxy carbonyl méthyl) thio résorcine.

6. Utilisation selon l'une quelconque des revendications précédentes, caractérisée par le fait que la concentration en dérivés de formule (I) dans la composition est comprise entre 0,01 % et 10 % en poids par rapport au poids total de la composition.

7. Utilisation selon l'une quelconque des revendications précédentes, caractérisée par le fait que la concentration en dérivés de formule (I) dans la composition est de préférence comprise entre 0,5 % et 5 % en poids par rapport au poids total de la composition.

8. Utilisation selon l'une quelconque des revendications précédentes caractérisée par le fait que la composition cosmétique ou dermatologique se présente sous forme d'une lotion, d'une crème, d'un lait, d'un gel, d'un masque, de microsphères ou nanosphères ou de dispersions vésiculaires.

9. Utilisation selon l'une quelconque des revendications précédentes, caractérisée par le fait que la composition cosmétique ou dermatologique contient en outre un humectant, un agent de surface, un kératolytique, un agent anti-inflammatoire, un agent complexant, un anti-oxydant, un conservateur, un parfum ou un filtre solaire.

## Claims

1. Use, as active compounds, of resorcinol derivatives for the preparation of a cosmetic or dermatological composition having a depigmenting action, characterized in that the said derivatives correspond to the following formula: 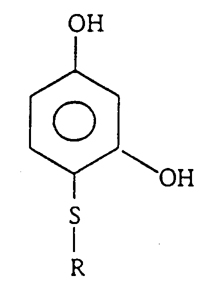 in which:
R represents a C₁-C₆ alkyl radical, a C₁-C₆ halogenoalkyl radical, an aryl radical, an aralkyl radical substituted or otherwise with a lower alkyl radical, a lower alkoxy or a halogen atom, the radical -CₙH₂ₙ-COOR' where n is an integer between 1 and 5 and R' represents a C₁-C₆ alkyl radical, a morpholino-alkyl or piperidino-alkyl radical whose alkyl radical is C₁-C₆, or the radical

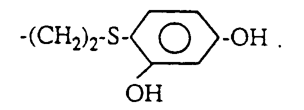

2. Use according to Claim 1, characterized in that the said aryl radical is the phenyl radical.

3. Use according to Claim 1, characterized in that the said aralkyl radical is the benzyl radical.

4. Use according to any one of the preceding claims, characterized in that the said derivatives of formula (I) are chosen from the group consisting of:
- 4-methylthioresorcinol,
- 4-ethylthioresorcinol,
- 4-n-butylthioresorcinol,
- 4-n-propylthioresorcinol,
- 4-isopropylthioresorcinol,
- 4-ethoxycarbonylmethylthioresorcinol,
- 4-ethoxycarbonylisopropylthioresorcinol,
- 4-benzylthioresorcinol,
- 4-phenylthioresorcinol,
- 4-(4'-methoxybenzyl)thioresorcinol,
- 4-(4'-chlorobenzyl)thioresorcinol,
- 4-(4'-methylbenzyl)thioresorcinol,
- 4-(3'-morpholinopropyl)thioresorcinol,
- 4-(4'-morpholinobutyl)thioresorcinol,
- 4-(4'-piperidinobutyl)thioresorcinol,
- 4-(4'-chlorobutyl)thioresorcinol,
- 4-(2',2'-dichloro-1',1',2'-trifluoroethyl)-thioresorcinol, and
- 1,2-bis(2',4'-dihydroxyphenyl)ethylene dithioether.

5. Use according to any one of the preceding claims, characterized in that the said derivatives of formula (I) are chosen from the group consisting of:
- 4-methylthioresorcinol,
- 4-ethylthioresorcinol,
- 4-n-propylthioresorcinol,
- 4-isopropylthioresorcinol,
- 4-benzylthioresorcinol,
- 1, 2-bis(2',4'-dihydroxyphenyl)ethylene dithioether,
- 4-(3'-morpholinopropyl)thioresorcinol, and
- 4-(ethoxycarbonylmethyl)thioresorcinol.

6. Use according to any one of the preceding claims, characterized in that the concentration of derivatives of formula (I) in the composition is between 0.01% and 10% by weight relative to the total weight of the composition.

7. Use according to any one of the preceding claims, characterized in that the concentration of derivatives of formula (I) in the composition is preferably between 0.5% and 5% by weight relative to the total weight of the composition.

8. Use according to any one of the preceding claims, characterized in that the cosmetic or dermatological composition is provided in the form of a lotion, a cream, a milk, a gel, a pack, microspheres or nanospheres or vesicular dispersions.

9. Use according to any one of the preceding claims, characterized in that the cosmetic or dermatological composition contains, in addition, a humectant, a surfactant, a keratolytic agent, an anti-inflammatory agent, a complexing agent, an antioxidant, a preservative, a perfume or a sunscreen.

## Patentansprüche

1. Verwendung von Resorcinderivaten als Wirkstoffe zur Herstellung einer kosmetischen oder dermatologischen Zubereitung mit einer dipigmentierenden Wirkung, dadurch **gekennzeichnet**, daß diese Derivate der folgenden Formel entsprechen: 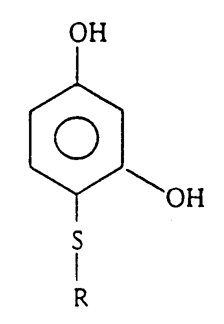 worin
R einen C₁-C₆-Alkylrest, einen C₁-C₆-Halogenalkylrest, einen Arylrest, einen gegebenenfalls durch einen niederen Alkylrest, einen niederen Alkoxyrest oder ein Halogenatom substituierten Aralkylrest, oder den Rest den -CₙH₂ₙ-COOR' bedeutet, worin n eine ganze Zahl von 1 bis 5 und
R' einen C₁-C₆-Alkylrest, einen Morpholinalkyl- oder Piperidinlyalkylrest darstellt, bei welchem der Alkylrest 1 bis 6 C-Atome aufweist oder die Gruppe bedeutet:

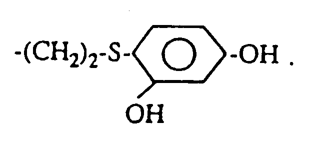

2. Verwendung nach Anspruch 1, dadurch **gekennzeichnet**, daß der Arylrest eine Phenylgruppe bedeutet.

3. Verwendung nach Anspruch 1, dadurch **gekennzeichnet**, daß der Aralkylrest die Benzylgruppe bedeutet.

4. Verwendung nach einem der vorstehenden Ansprüche, dadurch **gekennzeichnet**, daß die Derivate gemäß der Formel (I) aus der aus den folgenden Resten bestehenden Gruppe ausgewählt sind:
4-Methylthioresorcin,
4-Ethylthioresorcin,
4-n-Butylthioresorcin,
4-n-Propylthioresorcin,
4-Isopropylthioresorcin,
4-Ethoxycarbonylmethylthioresorcin,
4-Ethoxycarbonylisopropylthioresorcin,
4-Benzylthioresorcin,
4-Phenylthioresorcin,
4-(4'-Methoxybenzyl)-thioresorcin,
4-(4'-Chlorbenzyl)-thioresorcin,
4-(4'-Methylbenzyl)-thioresorcin,
4-(3'-Morpholinylpropyl)-thioresorcin,
4-(4'-Morpholinylbutyl)-thioresorcin,
4-(4'-piperidinylbutyl)-thioresorcin,
4-(4'-Chlorbutyl)-thioresorcin,
4-(2',2'-Dichlor-1',1',2'-trifluorethyl)-thioresorcin sowie den 1,2-Bis-(2',4'-dihydroxyphenyl)-ethylendithioether.

5. Verwendung nach einem der vorstehenden Ansprüche, dadurch **gekennzeichnet**, daß die Derivate gemäß der Formel (I) aus der aus den folgenden Resten bestehenden Gruppe ausgewählt sind:
4-Methylthioresorcin,
4-Ethylthioresorcin,
4-n-Propylthioresorcin,
4-Isopropylthioresorcin,
4-Benzylthioresorcin,
1,2-Bis-(2'4'-dihydroxyphenyl)-ethylendithioether,
4-(3'-Morpholinylpropyl)-thioresorcin,
4-(Ethoxycarbonylmethyl)-thioresorcin.

6. Verwendung nach einem der vorstehenden Ansprüche, dadurch **gekennzeichnet**, daß die Konzentration an Derivaten gemäß der Formel (I) in der Zubereitung zwischen 0,01 Gew.-% und 10 Gew.-% in bezug auf das Gesamtgewicht der Zusammensetzung beträgt.

7. Verwendung nach einem der vorstehenden Ansprüche, dadurch **gekennzeichnet**, daß die Konzentration an Derivaten gemäß der Formel (I) in der Zubereitung vorzugsweise zwischen 0,05 Gew.-% und 5 Gew.-% in bezug auf das Gesamtgewicht der Zusammensetzung beträgt.

8. Verwendung nach einem der vorstehenden Ansprüche, dadurch **gekennzeichnet**, daß die kosmetische oder dermatologische Zubereitung in Form einer Lotio, Creme, einer Milch, eines Gels, einer Maske, in Form von Mikro- oder Nanokügelchen oder Vesikeldispersionen vorliegt.

9. Verwendung nach einem der vorstehenden Ansprüche, dadurch **gekennzeichnet**, daß die kosmetische oder dermatologische Zubereitung zusätzlich noch ein Feuchthaltemittel, ein Tensid, ein keratolytisches Agens, einen Entzündungshemmer, ein Komplexierungsmittel, ein Antioxidans, ein Konservierungsmittel, einen Duftstoff oder eine Sonnenfiltersubstanz enthält.
